# EUROPÄISCHE PATENTSCHRIFT

(11) **EP 0 497 079 B1**
(45) Veröffentlichungstag und Bekanntmachung des Hinweises auf die Patenterteilung: **15.05.1996**
(21) Anmeldenummer: 91810973.7
(22) Anmeldetag: 13.12.1991
(51) Int. Cl.: A61F 2/38

(54) **Kniegelenkprothese**
Knee joint prosthesis
Prothèse d'articulation du genou

(30) Priorität: 18.01.1991 CH 144/91
(43) Veröffentlichungstag der Anmeldung: 05.08.1992
(73) Patentinhaber: SULZER Medizinaltechnik AG, CH-8404 Winterthur (CH)
(72) Erfinder: Koch, Rudolf, CH-8267 Berlingen (CH); Streicher, Robert Michael, CH-8405 Winterthur (CH)
(74) Vertreter: Trieblnig, Adolf

(56) Entgegenhaltungen:
- EP-A- 0 346 183
- DE-A- 2 933 174
- FR-A- 2 550 936
- GB-A- 1 527 498
- US-A- 4 340 978

## Beschreibung

Die Erfindung betrifft eine Kniegelenkprothese mit einem Femurteil, dessen doppelt konvex gekrümmte, kondylenartige Tragflächen in Richtung anterior/posterior aus mindestens zwei Kreissektoren mit unterschiedlichen Radien und Mittelpunkten gebildet sind und in Richtung medial/lateral je eine einzige Krümmung aufweisen, deren Radius dem grösseren Radius der Anterior/posterior-Krümmung entspricht, sowie mit einem Tibiateil, bei dem zwischen einem in der Tibia verankerbaren, metallenen Tibiaplateau und dem Femurteil ein Zwischenteil aus Kunststoff vorhanden ist, dessen dem Femur zugewandte Oberfläche doppelt konkav gekrümmte Lagerflächen für die Femurkondylen aufweist.

Kniegelenkprothesen der vorstehend beschriebenen Art sind vielfach bekannt; als Beispiele wird verwiesen auf die CH-A-674 798 oder die EP-B-0 021 421 (Büchel/Pappas Meniskusknie) aus der Patentfamilie der US-A-4,340,978. Bei diesen Prothesen hat die Praxis nun gezeigt, dass die Zwischenteile im Laufe der Zeit einen unzulässig hohen Abrieb erleiden, dessen Teilchen in dem das Gelenk umgebenden lebenden Gewebe zu unerwünschten Einschlüssen und damit zu Störungen und Schädigungen führen.

Aufgabe der Erfindung ist es, den Abrieb bei dem Kunststoff- Zwischenteil zu vermindern oder weitgehend zu vermeiden. Dieses Ziel wird durch die Kennzeichen vom unabhängigen Anspruch 1 erreicht.

Die Lagerflächen eines Zwischenteils sind auf diese Weise mit einer Anzahl von Auflagern versehen, die als abriebsvermindernde Tragflächen für den Femurteil wirken. Um bei den Auflagern eine möglichst grosse "Belastungsfläche" zu erreichen, sind deren Stützflächen komplementär an die doppelt konvex gekrümmten Tragflächen des Femurteils angeglichen, d.h. ebenfalls als Ausschnitte doppelt konkav gekrümmter Flächen ausgebildet. Um sowohl bei gestrecktem als auch bei flexiertem Knie eine möglichst grossflächige Kraftaufnahme der Stützflächen der harten Stützkörper zu erreichen, haben die Stützflächen zwei unterschiedliche konkave Krümmungen, deren Radien den Radien der konvexen Anterior/posterior-Krümmungen der Femurkondylen entsprechen, wobei bevorzugterweise die Randbereiche der Stützflächen einen dem grösseren Radius der Kondylen und die zentralen Bereiche einen dem kleineren Radius der Kondylen entsprechenden Radius haben. Aus fabrikatorischen Gründen werden dabei Stützflächen bevorzugt, die Ausschnitte aus Kugelflächen sind.

Um die "Einstellung" oder "Anpassung" der Stützkörper an die Tragflächen des Femurteils zu erleichtern, können die Auflageflächen, mit denen die Stützkörper in Vertiefungen des Zwischenteils gelagert sind, und/oder die Gegenflächen dazu - d.h. die "Böden" - in den Vertiefungen des Zwischenteils bombiert sein. Bei derartig bombierten Stützkörpern oder Zwischenteilen ist es zusätzlich vorteilhaft, wenn die Mantelflächen der Stützkörper und die dazu komplementären Gegenflächen der Vertiefungen im Zwischenteil als Ausschnitte aus Kugelflächen ausgebildet sind, da dadurch eine gewisse Führung der Stützkörperbewegungen im Zwischenteil erfolgt.

In bekannter Weise können die Zwischenteile auf dem Tibiaplateau unter Umständen schwimmend gelagert sein; bei derartigen Konstruktionen ist es möglicherweise vorteilhaft, wenn die dem Tibiaplateau zugewandte Oberfläche des Zwischenteils ebenfalls mit metallenen Stützkörpern bestückt ist, deren Stützflächen an die Form der Grenzfläche zwischen Tibiaplateau und Zwischenteil angepasst ist.

Schliesslich ist es sinnvoll, wenn die Stützkörper zumindest der gleichen Materialklasse angehören wie der Femurteil, d.h. bei keramischen Tragflächen des Femurteils aus Keramik und bei metallischen Tragflächen des Femurteils aus Metall bestehen; insbesondere ist es möglich, zumindest die Gleit- oder Stützflächen der Stützkörper aus dem gleichen Material zu fertigen wie die Tragflächen des Femurteils.

Im folgenden wird die Erfindung anhand eines Ausführungsbeispiels im Zusammenhang mit der Zeichnung näher erläutert.
- Fig. 1: zeigt, teilweise im Schnitt I-I von Fig. 2, eine Ansicht der neuen Kniegelenkprothese von medial oder lateral aus gesehen;
- Fig. 2: ist eine Aufsicht auf einen Zwischenteil einer Prothese nach Fig. 1;
- Fig. 3: gibt einen Schnitt III-III von Fig. 2 wieder, während
- Fig. 4: in vergrössertem Massstab ein Detail aus Fig. 1 zeigt.

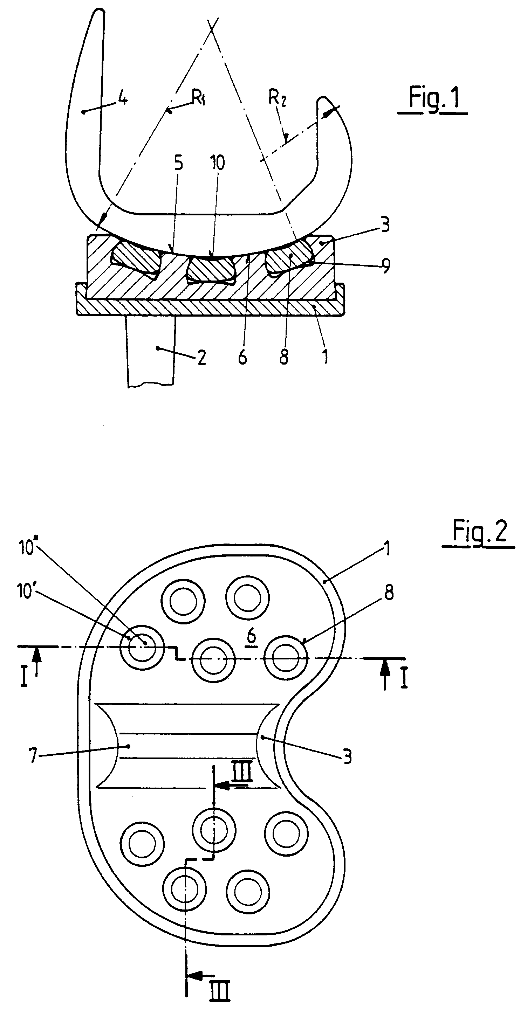

Auf einem Tibiaplateau 1 (Fig. 1), das mittels eines zapfenartigen Schaftes 2 in der nicht dargestellten Tibia verankerbar ist, ist ein Zwischenteil 3 gelagert, das - um zwischen dem im allgemeinen metallenen Tibiaplateau 1 und einem nur schematisch angedeuteten, ebenfalls meist aus Metall gefertigten Femurteil 4 als stossdämpfendes elastisches Element zu wirken - aus einem, unter Umständen faserverstärkten, Kunststoff, beispielsweise aus Polyethylen (PE), besteht.

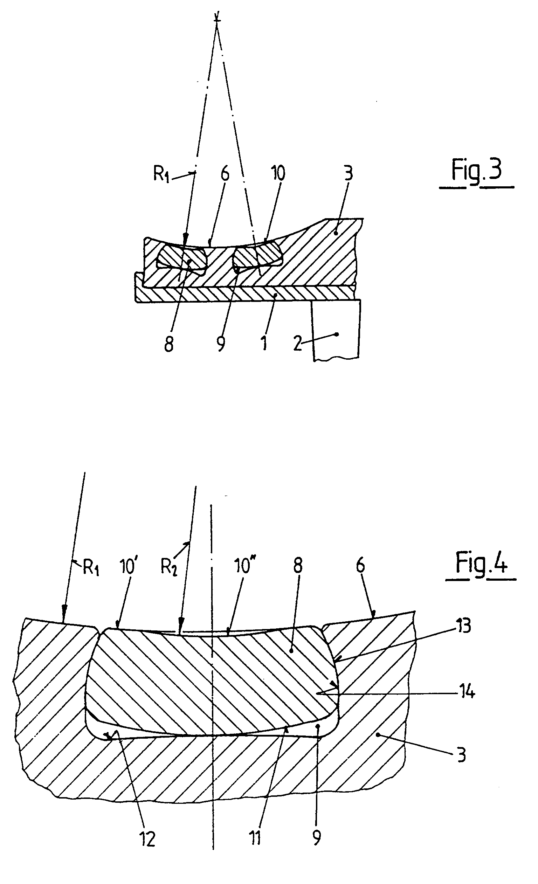

Die Tragflächen 5 des Femurteils 4 sind doppelt konvex gekrümmte Flächen, wie aus Fig. 1 und für die dazu komplementäre konkave Lagerfläche 6 aus Fig. 3 ersichtlich; in medial/lateraler Richtung ist bei jeder Kondyle eine einzige Krümmung vorhanden, deren Krümmungsradius mit R1 bezeichnet ist, wie wieder für die zughörige Lagerfläche 6 aus Fig. 3 zu erkennen ist. Die Krümmung der Tragflächen 5 des Femurteils 4 in Richtung anterior/posterior setzt sich aus zwei Kreissektoren zusammen, von denen der eine - der in der dargestellten Standphase des Gelenkes "tragende" - ebenfalls den Radius R1 hat; an den ersten Kreissektor schliesst sich posterior ein Kreissektor mit dem kleineren Krümmungsradius R2 an. Der Bereich der Tragflächen 5 mit dem kleineren Krümmungsradius R2 kommt bei abgewinkeltem Knie zum "Einsatz".

Für jede Femurkondyle ist im Zwischenteil 3 eine Lagerfläche 6 vorgesehen, die einen Ausschnitt aus einer Hohlkugelfläche mit dem Radius R1 darstellt. Zur seitlichen Führung des Femurteil 4 ist der Zwischenteil 3 zwischen den Lagerflächen 6 für die beiden Kondylen mit einer wulstartigen Erhöhung 7 versehen (Fig. 2).

In jeder der Lagerflächen 6 sind als tragende Elemente Stützkörper 8 vorhanden , die in Vertiefungen 9 des Zwischenteils 3 derart eingesetzt sind, dass ihre Stützflächen 10 möglichst genau in den Lagerflächen 6 liegen. Zumindest die Stützflächen der Stützkörper 8 bestehen aus einem relativ zum Kunststoff des Zwischenteils 3 harten Material, das zumindest der gleichen Materialklasse angehört oder, vorzugsweise, das Gleiche ist wie dasjenige der Tragflächen 5 des Femurteils 4.

Die Stützflächen 10, die möglichst exakt in die Lagerfläche 6 des Zwischenteils 3 integriert sind, sind in ihrer Form an die Tragflächen 5 des Femurteils 4 komplementär angepasst. Sie sind daher als Ausschnitte aus Kugelflächen ausgebildet, wobei die Randbereiche 10' (Fig. 2 und 4) einen Radius aufweisen, der dem Radius R1 der Anterior/posterior-Krümmung der Kondylentragflächen 5 in der Standphase entspricht, während die Zentralbereiche 10'' (Fig. 2 und 4) jeder Stützfläche 10 den Radius R2 des posterioren Tragflächenbereiches haben. Auf diese Weise schmiegen sich die Stützflächen 10 bei gestrecktem und bei abgewinkeltem Knie gut an die Tragflächen 5 des Femurteils 4 an, und die Belastungen werden über eine relativ grosse Gesamtfläche verteilt weitergeleitet.

Sowohl die Auflageflächen 11 (Fig. 4), mit denen die Stützkörper 8 auf den Gegenflächen oder "Böden" 12 (Fig. 4) der Vertiefungen 9 des Zwischenteils 3 gelagert sind, als auch diese Gegenflächen 12 selbst sind bombiert ausgebildet. Damit werden die Relativbewegungen der Stützkörper 8 gegenüber dem Zwischenteil 3, und damit das Anliegen der Stützflächen 10 an den Tragflächen 5 erleichtert. Um diese Relativbewegungen zu führen, sind sowohl die Mantelflächen 13 (Fig. 4) der Stützkörper 8, als auch die dazu komplementären Flächen 14 (Fig. 4) der Vertiefungen 9 als Kugel- bzw. Hohlkugelflächen ausgeführt.

Da, wie bereits erwähnt, der Femurteil 4 und seine Tragflächen 5 vorzugsweise aus Metall bestehen, sind die Stützkörper 8 in den meisten Anwendungsfällen ebenfalls aus Metall, beispielsweise einer Kobaltbasislegierung oder aus einer anderen der für Implantate gebräuchlichen Legierungen gefertigt.

## Patentansprüche

1. Kniegelenkprothese mit einem Femurteil (4), dessen doppelt konvex gekrümmten, kondylenartigen Tragflächen (5) in Richtung anterior/posterior aus mindestens zwei Kreissektoren mit unterschiedlichen Radien (R1 und R2) und Mittelpunkten gebildet sind und in Richtung medial/lateral je eine einzige Krümmung aufweisen, deren Radius dem grösseren Radius (R1) der Anterior/posterior-Krümmung entspricht, sowie mit einem Tibiateil, bei dem zwischen einem in der Tibia verankerbaren, metallenen Tibiaplateau (1) und dem Femurteil(4) ein Zwischenteil (3) aus Kunststoff vorhanden ist, dessen dem Femur zugewandte Oberfläche doppelt konkav gekrümmte Lagerflächen (6) für die Femurkondylen aufweist, **dadurch gekennzeichnet,** dass in den Lagerflächen (6) des Zwischenteils (3) einzelne Stützkörper (8) aus, relativ zum Kunststoff, hartem Material verteilt sind, deren in der Lagerfläche (6) liegende Stützflächen (10) ebenfalls doppelt konkav gekrümmt sind und dass die Stützflächen (10) zwei unterschiedliche konkave Krümmungen haben, deren Radien den Radien (R1 und R2) der konvexen Anterior/posterior-Krümmungen der Femurkondylen entsprechen.

2. Kniegelenkprothese nach Anspruch 1, dadurch gekennzeichnet, dass die Stützflächen (10) Ausschnitte aus Kugelflächen sind.

3. Kniegelenkprothese nach Anspruch 2, dadurch gekennzeichnet, dass die Randbereiche (10') der Stützflächen (10) einen dem grösseren Radius (R1) der Kondylen und die zentralen Bereiche (10'') einen dem kleineren Radius (R2) der Kondylen entsprechenden Radius haben.

4. Kniegelenkprothese nach einem der Ansprüche 1 bis 3, dadurch gekennzeichnet, dass die Auflageflächen (11), über die die Stützkörper (8) in Vertiefungen (9) des Zwischenteils (3) gelagert sind, bombiert sind.

5. Kniegelenkprothese nach Anspruch 4, dadurch gekennzeichnet, dass in den Vertiefungen (9) des Zwischenteils (3) die Gegenflächen (12) zu den Auflageflächen (11) der Stützkörper (8) ebenfalls bombiert sind.

6. Kniegelenkprothese nach einem der Ansprüche 1 bis 5, dadurch gekennzeichnet, dass der Zwischenteil (3) schwimmend auf dem Tibiaplateau (1) gelagert ist.

7. Kniegelenkprothese nach Anspruch 6, dadurch gekennzeichnet, dass die dem Tibiaplateau (1) zugewandte Oberfläche des Zwischenteils (3) ebenfalls mit metallenen Stützkörpern bestückt ist, deren Stützflächen an die Form der Grenzfläche zwischen Tibiaplateau (1) und dem Zwischenteil (3) angepasst ist.

8. Kniegelenkprothese einem der Ansprüche 1 bis 5, dadurch gekennzeichnet, dass die Mantelflächen (13) der Stützkörper (8) und die dazu komplementären Flächen (14) der Vertiefungen (9) des Zwischenteils (3) als Ausschnitte aus Kugelflächen ausgebildet sind.

9. Kniegelenkprothese nach einem der vorhergehenden Ansprüche, dadurch gekennzeichnet, dass die Stützkörper (8) der gleichen Materialklasse angehören wie der Femurteil (4).

10. Kniegelenkprothese nach einem der vorhergehenden Ansprüche, dadurch gekennzeichnet, dass die Stützkörper (8) aus dem gleichen Material wie der Femurteil (4) bestehen.

## Claims

1. A knee joint prosthesis having a femur part (4), in which condylar load-bearing surfaces (5), having a double convex curve, are formed in the anterior/posterior direction from at least two circular sectors having different radii (R1 and R2) and centre points, and in the medial/lateral direction each have a single curve, the radius of which corresponds to the larger radius (R1) of the anterior/posterior curve, and also having a tibia part, in which there is an intermediate part (3) made of plastics between a metal tibia plate (1), which can be secured in the tibia, and the femur part (4), in which part the surface facing the femur has bearing surfaces (6), which have a double concave curve, for the femur condyles,
**characterised in that** individual support members (8), made from a material which is relatively hard in comparison with the plastic, are distributed in the bearing surfaces (6) of the intermediate part (3), in which the support surfaces (10), which lie in the bearing surface (6), also have double concave curves, and in that the support surfaces (10) have two different concave curves, the radii of which correspond to the radii (R1 and R2) of the convex anterior/posterior curves of the femur condyles.

2. A knee joint prosthesis according to Claim 1,
**characterised in that** the support surfaces (10) are segments from spherical surfaces.

3. A knee joint prosthesis according to Claim 2,
**characterised in that** the edge regions (10') of the support surfaces (10) have a radius corresponding to the larger radius (R2) of the condyles and the central regions (10'') have a radius corresponding to the smaller radius (R2) of the condyles.

4. A knee joint prosthesis according to one of Claims 1 to 3,
**characterised in that** the contact surfaces (11), above which the support members (8) are mounted in depressions (9) in the intermediate part (3), are dished.

5. A knee joint prosthesis according to Claim 4,
**characterised in that** in the depressions (9) of the intermediate part (3) the counter-surfaces (12) to the contact surfaces (11) of the support members (8) are also dished.

6. A knee joint prosthesis according to one of Claims 1 to 5,
**characterised in that** the intermediate part (3) is mounted so that it floats on the tibia plate (1).

7. A knee joint prosthesis according to Claim 6,
**characterised in that** the surface of the intermediate part (3) facing the tibia plate (1) is also covered with metal support members, the support surfaces of which is adapted to the shape of the boundary surface between the tibia plate (1) and the intermediate part (3).

8. A knee joint prosthesis according to one of Claims 1 to 5,
**characterised in that** the shell surfaces (13) of the support members (8) and the surfaces (14) of the depressions (9) of the intermediate part (3) complementary thereto are constructed as segments from spherical surfaces.

9. A knee joint prosthesis according to one of the preceding Claims,
**characterised in that** the support members (8) belong to the same material class as the femur part (4).

10. A knee joint prosthesis according to one of the preceding Claims,
**characterised in that** the support members (8) are made from the same material as the femur part (4).

## Revendications

1. Prothèse d'articulation du genou, comprenant une partie fémorale (4) dont les surfaces portantes (5) en forme de condyles à double courbure convexe sont formées, dans la direction antéro-postérieure, d'au moins deux secteurs circulaires possédant des rayons (R1 et R2) et des centres différents et présentent chacune, dans la direction médio-latérale, une courbure unique dont le rayon correspond au plus grand rayon (R1) de la courbure antéro-postérieure, et comprenant une partie tibiale dans laquelle est prévu, entre un plateau tibial métallique (1) ancré dans le tibia et la partie fémorale (4), une pièce intermédiaire (3) en matière synthétique, dont la surface tournée vers le fémur comporte des surfaces d'appui (6) à double courbure concave destinées aux condyles fémoraux, caractérisée en ce que, dans les surfaces d'appui (6) de la pièce intermédiaire (3), sont répartis des corps de soutien isolés (8) qui sont réalisés en matériau dur par rapport à la matière synthétique et dont les surfaces de soutien (10) situées dans la surface d'appui (6) présentent elles aussi une double courbure concave, et en ce que les surfaces de soutien (10) présentent deux courbures concaves différentes dont les rayons correspondent aux rayons (R1 et R2) des courbures antéro-postérieures convexes des condyles fémoraux.

2. Prothèse d'articulation du genou selon la revendication 1, caractérisée en ce que les surfaces de soutien (10) sont des segments de surfaces sphériques.

3. Prothèse d'articulation du genou selon la revendication 2, caractérisée en ce que les zones de bord (10') des surfaces de soutien (10) possèdent un rayon correspondant au plus grand rayon (R1) des condyles et les zones centrales (10'') possèdent un rayon correspondant au plus petit rayon (R2) des condyles.

4. Prothèse d'articulation du genou selon l'une des revendications 1 à 3, caractérisée en ce que les surfaces de contact (11), au moyen desquelles les corps de soutien (8) reposent dans des alvéoles (9) de la pièce intermédiaire (3), sont bombées.

5. Prothèse d'articulation du genou selon la revendication 4, caractérisée en ce que, dans les alvéoles (9) de la pièce intermédiaire (3), les surfaces antagonistes (12) des surfaces de contact (11) des corps de soutien (8) sont elles aussi bombées.

6. Prothèse d'articulation du genou selon l'une des revendications 1 à 5, caractérisée en ce que la pièce intermédiaire (3) est montée flottante sur le plateau tibial (1).

7. Prothèse d'articulation du genou selon la revendication 6, caractérisée en ce que la surface de la pièce intermédiaire (3) tournée vers le plateau tibial (1) est également garnie de corps de soutien métalliques dont les surfaces de soutien épousent la forme de la surface de séparation entre le plateau tibial (1) et la pièce intermédiaire (3).

8. Prothèse d'articulation du genou selon l'une des revendications 1 à 5, caractérisée en ce que les surfaces périphériques (13) des corps de soutien (8) et les surfaces complémentaires correspondantes (14) des alvéoles (9) de la pièce intermédiaire (3) sont conçues sous la forme de segments de surfaces sphériques.

9. Prothèse d'articulation du genou selon l'une des revendications précédentes, caractérisée en ce que les corps de soutien (8) appartiennent à la même classe de matériaux que la partie fémorale (4).

10. Prothèse d'articulation du genou selon l'une des revendications précédentes, caractérisée en ce que les corps de soutien (8) sont réalisés dans le même matériau que la partie fémorale (4).
